(19) Europäisches Patentamt
European Patent Office
Office européen des brevets

(11) Publication number: **0 046 330 A2**

(12) **EUROPEAN PATENT APPLICATION**

(21) Application number: **81302657.2**

(22) Date of filing: **15.06.81**

(51) Int. Cl.³: **C 07 F 15/04**, B 01 J 31/28, C 07 C 2/08

(30) Priority: **18.08.80 US 179075**

(43) Date of publication of application: **24.02.82 Bulletin 82/8**

(84) Designated Contracting States: **DE FR GB IT**

(71) Applicant: **GULF RESEARCH & DEVELOPMENT COMPANY, P.O. Box 2038, Pittsburgh Pennsylvania 15230 (US)**

(72) Inventor: **Beach, David L., 1529 Mountain View Drive, Gibsonia Pennsylvania 15044 (US)**
Inventor: **Harrison, James J., 211 North Rose Drive, Glenshaw Pennsylvania 15116 (US)**

(74) Representative: **Huskisson, Frank Mackie et al, Fitzpatricks 48 St. Vincent Street, Glasgow, G2 5TT Scotland (GB)**

(54) **Process for the preparation of nickel ylides containing sulfonated group V ligands.**

(57) A process is provided for preparing nickel ylides wich are themselves novel compounds defined by the following Formula I:

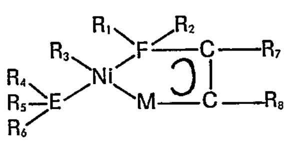

wherein $R_1$, $R_2$, $R_3$, $R_4$, $R_5$, $R_6$, $R_7$ and $R_8$ are either alike or different members selected from the group consisting of hydrogen, alkyl radicals having from about one to about 24 carbon atoms, preferably from about one to about 10 carbon atoms; aryl radicals having from about six to about 20 carbon atoms, preferably from about six to about 10 carbon atoms; alkenyl radicals having from about two to about 30 carbon atoms, preferably from about two to about 20 carbon atoms; cycloalkyl radicals having from about three to about 40 carbon atoms, preferably from about three to about 30 carbon atoms; aralkyl and alkaryl radicals having from about six to about 40 carbon atoms, preferably from about six to about 30 carbon atoms; a halogen radical selected from the group consisting of fluorine, chlorine, bromine and iodine, preferably chlorine; a hydroxyl group; an alkoxy or aryloxy group; a hydrocarbyl group, such as defined above, carrying halogen, hydroxyl or alkoxy or aryloxy; and a sulfonato group ($-SO_3^-$) or an alkyl, aryl, alkenyl, cycloalkyl, aralkyl or alkaryl group carrying a sulfonato group; provided that at least one sulfonato group is located in $R_4$, $R_5$ and $R_6$ and at least one of $R_4$, $R_5$ and $R_6$ is aryl; M is sulfur or oxygen, preferably oxygen; E is phosphorus, arsenic, antimony or nitrogen, preferably phosphorus; and F is phosphorus, arsenic or antimony, preferably phosphorus. The process comprises sulfonating a ligand defined by the following formula:

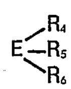

and then reacting the resulting sulfonated ligand with (1) a zero valent nickel compound and (2) a ylide defined by the following formula:

EP 0 046 330 A2

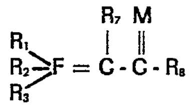

wherein $R_1$, $R_2$, $R_3$, $R_4$, $R_5$, $R_6$, $R_7$, $R_8$, E, F and M are as defined above, provided that at least one of $R_4$, $R_5$ and $R_6$ is an aryl group.

# PROCESS FOR THE PREPARATION OF NICKEL YLIDES CONTAINING SULFONATED GROUP V LIGANDS

## Cross-References to Related Applications

Reference is made to applicants' following U.S. applications:

U.S. Patent application Serial No. 179079, filed 18 August 1980, entitled "Nickel Ylides".

U.S. Patent application Serial No. 179080, filed 18 August 1980, entitled "Process for the Preparation of Nickel Ylides Containing Ylide Ligands with a Sulfonated Group V Component".

U.S. Patent application Serial No. 179078, filed 18 August 1980 entitled "Process for the Preparation of Nickel Ylides Containing Directly Sulfonated Ylide Ligands".

U.S. Patent application Serial No. 179076, filed 18 August 1980 entitled "Process for the Oligomerization of Ethylene".

U.S. Patent application Serial No. 179005, filed 18 August 1980, entitled "Process for the Oligomerization of Ethylene in Methanol."

The disclosures of the foregoing applications are hereby incorporated by reference.

## Field Of The Invention

The present invention relates to a novel process for preparing nickel ylides which are useful as catalysts for the oligomerization of ethylene.

## Description Of The Prior Art

It is well known in the art to use a variety of catalysts to oligomerize ethylene to higher molecular weight olefins. The term "oligomerize" has been employed, and is employed herein to describe the conversion of lower olefins such as ethylene to olefinic products of higher molecular weight, e.g., to dimer, trimer, tetramer and the like. The reaction rate and product distribution obtained are highly dependent on the exact catalyst composition and the reaction conditions employed. Two such general classes of catalysts are the "Ziegler" types consisting of aluminum trialkyls and the "Ziegler-Natta" types consisting of aluminum alkyls or alkyl halides and titanium halides. Major disadvantages of aluminum alkyl catalysts are their highly reactive and pyrophoric nature and the fact that they must be used at relatively high temperature, e.g., 200-275°C. and pressures, e.g., 2000-4000 psig (13,790 to 27,580 kPa). Although much milder reaction conditions are used when the aluminum alkyls are used in conjunction with titanium halides, product quality and ease of catalyst separation from products of both of these prior art types of catalysts are not as high as desired.

An article by W. Keim, F.H. Kowaldt, R. Goddard and C. Kruger entitled "Novel Coordination of (Benzoylmethylene)triphenylphosphorane in a Nickel Oligomerization Catalyst", in Angew. Chem. Int. Ed. Engl. (1978) No. 6, page 466, discloses the preparation of a nickel ylide by the following reaction:

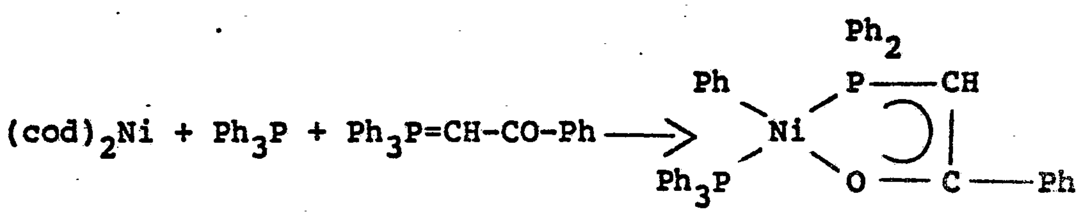

wherein "cod" represents 1,5-cyclooctadiene and "Ph" represents phenyl. It is reported that the resultant nickel ylide converts ethylene into alpha olefins or polyethylene.

Summary Of The Invention

A novel process has now been found for preparing nickel ylides which are themselves novel compounds defined by the following Formula I:

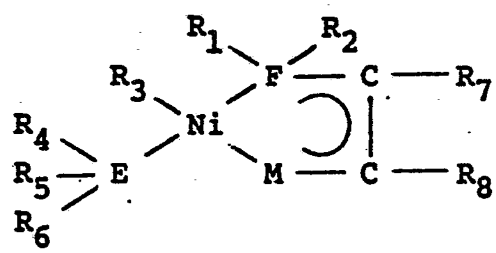

wherein $R_1$, $R_2$, $R_3$, $R_4$, $R_5$, $R_6$, $R_7$ and $R_8$ are either alike or different members selected from the group consisting of hydrogen, alkyl radicals having from about one to about 24 carbon atoms, preferably from about one to about 10 carbon atoms; aryl radicals having from about six to about 20 carbon atoms, preferably from about six to about 10 carbon atoms; alkenyl radicals having from about two to about 30 carbons atoms, preferably from about two to about 20 carbon atoms; cycloalkyl radicals having from about three to about 40 carbon atoms, preferably from about three to about 30 carbon atoms; aralkyl and alkaryl radicals having from about six to about 40 carbon atoms, preferably from about six to about 30 carbon atoms; a halogen radical

selected from the group consisting of fluorine, chlorine, bromine and iodine, preferably chlorine; a hydroxyl group; an alkoxy or aryloxy group; a hydrocarbyl group, such as defined above, carrying halogen, hydroxyl or alkoxy or aryloxy; and a sulfonato group ($-SO_3^-$) or an alkyl, aryl, alkenyl, cycloalkyl, aralkyl or alkaryl group carrying a sulfonato group; provided that at least one sulfonato group is located in $R_4$, $R_5$ and $R_6$ and at least one of $R_4$, $R_5$ and $R_6$ is aryl; M is sulfur or oxygen, preferably oxygen; E is phosphorus, arsenic, antimony or nitrogen, preferably phosphorus; and F is phosphorus, arsenic or antimony, preferably phosphorus. The process comprises sulfonating a ligand defined by the following formula:

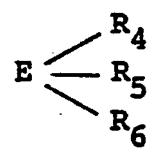

and then reacting the resulting sulfonated ligand with (1) a zero valent nickel compound and (2) a ylide defined by the following formula:

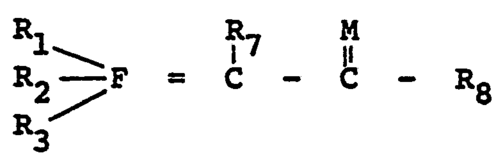

wherein $R_1$, $R_2$, $R_3$, $R_4$, $R_5$, $R_6$, $R_7$, $R_8$, E, F and M are as defined above, provided that at least one of $R_4$, $R_5$ and $R_6$ is an aryl group.

The presence of the sulfonato group in the nickel ylides obtained in the process of this invention induces solubility in polar solvents such as water or methanol. This facilitates product removal and separation from reaction media or the use of extractive techniques, e.g., by the use of aqueous ammonium hydroxide, not

possible with the corresponding nickel ylides which do not contain a sulfonato group.

Description Of The Preferred Embodiments

The first step in the process of this invention involves sulfonating a ligand defined by the formula:

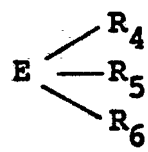

wherein $R_4$ to $R_6$ and E are as defined above, provided that at least one of $R_4$, $R_5$ and $R_6$ is an aryl group as defined above using $SO_3$ in the presence of a strong inorganic mineral acid, such as sulfuric acid, hydrochloric acid, nitric acid, phosphoric acid, etc. Specific examples of such ligands that can be used include: allyldiphenylphosphine; benzyldiphenylphosphine; bis(3-aminopropyl)phenylphosphine; bis(2-cyanoethyl)phenylphosphine; bis(m-fluorophenyl)phosphinous chloride; 4-bromophenyldiphenylphosphine; n-butyldiphenylphosphine; t-butyldiphenylphosphine; 2-cyanoethyldiphenylphosphine; cyclohexyldiphenylphosphine; n-decylphenylphosphine; diallylphenylphosphine; di-n-amylphenylphosphine; di-sec-butylphenylphosphine; dicyclohexylphenylphosphine; diethylphenylphosphine; di-n-heptylphenylphosphine; di-n-hexylphenylphosphine; dimethylphenylphosphine; dimethyl-p-tolylphosphine; diphenyl-n-butoxyphosphine; diphenylchlorophosphine; diphenylenephenylphosphine; diphenylethoxyphosphine; diphenylmethoxyphosphine; diphenylphosphine; beta-diphenylphosphinoethyltriethoxysilane; di-iso-propylphenylphosphine; di-o-tolylphenylphosphine; divinylphenylphosphine; ethyldiphenylphosphine; n-hexyldiphenylphosphine; o-methoxyphenyldiphenylphosphine; (2-methylbutyl)diphenyl-

phosphine; methyldiphenylphosphine; methylethylphenylphosphine; methylphenylphosphine; neomenthyldiphenylphosphine; pentafluorophenyldiphenylphosphine; (2-phenylbutyl)diphenylphosphine; phenyldi-n-butoxyphosphine; phenyldichlorophosphine; phenyldiethoxyphosphine; phenyldimethoxyphosphine; phenylphosphine; isopropyldiphenylphosphine; n-propyldiphenylphosphine; o-tolyldiphenylphosphine; p-tolyldiphenylphosphine; tribenzylphosphine; tris(m-chlorophenyl)phosphine; tris(p-chlorophenyl)phosphine; tri(1-naphthyl)phosphine; triphenylphosphine; tris(4-dimethylaminophenyl)phosphine; tris(p-fluorophenyl)phosphine; tris(o-methoxyphenyl)phosphine; tris(p-methoxyphenyl)phosphine; tri-o-tolylphosphine; tri-m-tolylphosphine; tri-p-tolylphosphine; vinyldiphenylphosphine; sodium diphenylphosphinebenzene-3-sulfonate; disodium phenylphosphinebis(benzene-3-sulfonate); dimethylphenylarsine; methyldiphenylarsine; triphenylarsine; tri-p-tolylarsine; diphenylchloroarsine; triphenylantimony; triphenylamine; tribenzylamine; methyldiphenylamine; and dimethylphenylamine.

It is preferred to use fuming sulfuric acid ($H_2SO_4 \cdot x\ SO_3$, where x can be, for example, from about 0.1 to about 0.6, preferably from about 0.2 to about 0.4). The amount of $SO_3$ is not critical and can vary over a wide range, for example, at least about one mole per mole of ligand, preferably from about two to about 20 moles per mole of ligand. The two reactants are stirred and heated at a temperature of about 0° to about 200°C., preferably about 40° to about 100°C., for about one minute to about 48 hours, preferably for about 30 minutes to about four hours. Any suitable pressure can be used, although atmospheric pressure is preferred. At the end of this period the reactor contents are cooled to a temperature of about -30° to about 50°C., preferably about room temperature (about 26°C.), after which sufficient water and a suitable

base, such as an alkaline metal hydroxide, an alkali metal alkoxide, ammonium hydroxide, a hydrocarbyl-substituted ammonium hydroxide, etc. are added thereto to crystallize the sulfonated ligand out of solution. For example, the amount of water used can range from about 10 milliliters to about 10 liters per mole of sulfonated ligand. The crystals can be recovered in any suitable manner, for example, by filtration, decantation or by centrifuging.

In the second step of this process, the sulfonated ligand obtained in the first step is reacted with any zero valent nickel compound, or any nickel compound convertible to a zero valent nickel compound in situ, and a ylide defined by the following Formula II:

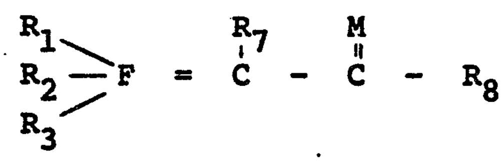

wherein $R_1$, $R_2$, $R_3$, $R_7$, $R_8$, M and F are as defined above. Specific examples of such nickel compounds which can be used include: tris(triphenylphosphine)nickel; bis(cyclooctadiene)nickel; tetrakis(triphenylphosphine)nickel; bis(norbornadiene)nickel; (cycloocta-1,5-diene)duroquinone nickel; (dicyclopentadiene)duroquinone nickel; bis(tetracyclone)nickel; tetrakis(triethylphosphine)nickel; tris(triethylphosphine)nickel; bis(triphenylphosphine)nickel dicarbonyl; nickel carbonyl; nickel(II)acetylacetonate; nickelocene; bis(triethylphosphine)nickel(II)chloride; tetrakis(trifluorophosphine)nickel; nickel acetate; nickel bromide; nickel carbonate; nickel chloride; nickel fluoride; nickel iodide; nickel nitrate; nickel sulfate; nickel 2,4-pentanedionate; bis $\pi$ -allyl nickel; and nickel dichloride hexaamine. Specific examples of ylides coming within the definition of Formula II are set forth in Table I. In this table and as used elsewhere herein, "Ph" represents phenyl and "Et" represents ethyl.

TABLE I

| Compound | $R_1$ | $R_2$ | $R_3$ | $R_7$ | $R_8$ | F | M |
|---|---|---|---|---|---|---|---|
| 1 | Ph | Ph | Ph | H | Ph | P | O |
| 2 | Ph | Ph | Ph | H | Ph | P | S |
| 3 | Ph | Ph | Ph | $SO_3^-$ | Ph | P | O |
| 4 | Ph | Ph | Ph | $SO_3^-$ | Ph | P | S |
| 5 | 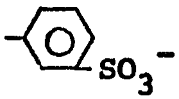 | Ph | Ph | H | Ph | P | O |
| 6 | Ph | 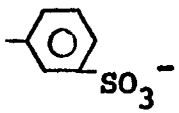 | Ph | $SO_3^-$ | Ph | P | O |
| 7 | Ph | Ph | Ph | H | $OCH_3$ | P | O |
| 8 | Ph | Ph | Ph | $SO_3^-$ | $OCH_3$ | P | O |
| 9 | Ph | Ph | Ph | H | $OCH_3$ | P | S |
| 10 | 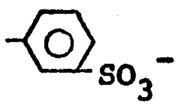 | Ph | Ph | H | $OCH_3$ | P | O |
| 11 | Ph | 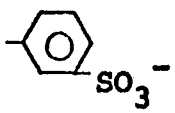 | Ph | $SO_3^-$ | $OCH_3$ | P | O |
| 12 | Ph | Ph | Ph | H | $CH_3$ | P | O |
| 13 | Ph | Ph | Ph | $SO_3^-$ | $CH_3$ | P | O |
| 14 | 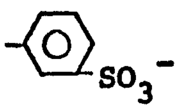 | Ph | Ph | H | $CH_3$ | P | O |

TABLE I (continued)

| Compound | $R_1$ | $R_2$ | $R_3$ | $R_7$ | $R_8$ | F | M |
|---|---|---|---|---|---|---|---|
| 15 | Ph | 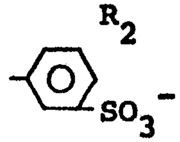 | Ph | $SO_3^-$ | $CH_3$ | P | O |
| 16 | Ph | Ph | Ph | $SO_3^-$ | $CH_3$ | P | S |
| 17 | Ph | Ph | Ph | H | $CH_3$ | P | S |
| 18 | $CH_3$ | $CH_3$ | $CH_3$ | H | Ph | P | O |
| 19 | $CH_3$ | $CH_3$ | $CH_3$ | $SO_3^-$ | Ph | P | O |
| 20 | $CH_3$ | $CH_3$ | $CH_3$ | H | Ph | P | S |
| 21 | $CH_3$ | $CH_3$ | $CH_3$ | $SO_3^-$ | Ph | P | S |
| 22 | Et | Et | Et | $SO_3^-$ | Ph | P | O |
| 23 | $CH_3$ | 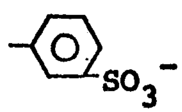 | Et | H | Ph | P | O |
| 24 | $CH_3$ | Cyclo-hexyl | Ph | $SO_3^-$ | Ph | P | S |
| 25 | $CH_3$ | $CH_3$ | $CH_3$ | H | $OCH_3$ | P | O |
| 26 | $CH_3$ | Ph | Et | $SO_3^-$ | $OCH_3$ | P | O |
| 27 | 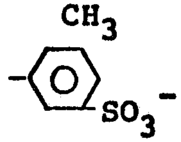 | Ph | Et | H | $OCH_3$ | P | S |
| 28 | $CH_3$ | $CH_3$ | $CH_3$ | H | $CH_3$ | P | O |

TABLE I (continued)

| Compound | $R_1$ | $R_2$ | $R_3$ | $R_7$ | $R_8$ | F | M |
|---|---|---|---|---|---|---|---|
| 29 | Ph | $CH_3$ | Et | $SO_3^-$ | $CH_3$ | P | O |
| 30 | Ph | 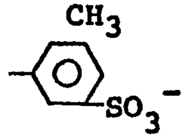 | $CH_3$ | H | $CH_3$ | P | S |
| 31 | Ph | Ph | Et | $SO_3^-$ | $CH_3$ | P | S |
| 32 | 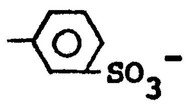 | $CH_3$ | Et | $SO_3^-$ | $CH_3$ | P | O |
| 33 | 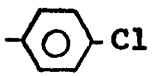 | Ph | Ph | $SO_3^-$ | Ph | P | O |
| 34 | 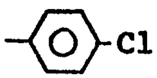 | Ph | $CH_3$ | H | Ph | P | S |
| 35 | $CH_3$ | $CH_3$ | Et | $SO_3^-$ | 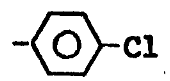 | P | O |
| 36 | Ph | Ph | Ph | H | Ph | As | O |
| 37 | Ph | Ph | Ph | H | Ph | As | S |
| 38 | Ph | Ph | Ph | $SO_3^-$ | Ph | As | O |
| 39 | Ph | Ph | Ph | $SO_3^-$ | $CH_3$ | As | O |
| 40 | $CH_3$ | $CH_3$ | $CH_3$ | H | Ph | As | O |
| 41 | Ph | $CH_3$ | $CH_3$ | $SO_3^-$ | Ph | As | O |
| 42 | Ph | 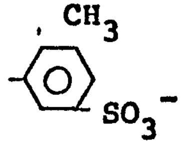 | $CH_3$ | H | Ph | As | O |

TABLE I (continued)

| Compound | $R_1$ | $R_2$ | $R_3$ | $R_7$ | $R_8$ | F | M |
|---|---|---|---|---|---|---|---|
| 43 | Ph | Ph | Ph | H | Ph | Sb | O |
| 44 | Ph | Ph | Ph | $SO_3^-$ | Ph | Sb | O |
| 45 | Ph | 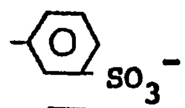 | Ph | H | Ph | Sb | O |
| 46 | Ph | 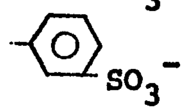 | Ph | $SO_3^-$ | Ph | Sb | O |
| 47 | Ph | Ph | Ph | H | Ph | Sb | S |
| 48 | Ph | Ph | Ph | $SO_3^-$ | Ph | Sb | S |
| 49 | $CH_3$ | $CH_3$ | $CH_3$ | H | Ph | Sb | O |
| 50 | $CH_3$ | Ph | $CH_3$ | $SO_3^-$ | Ph | Sb | O |
| 51 | Ph | Ph | Ph | H | 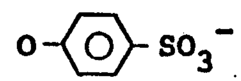 | P | O |
| 52 | Ph | Ph | Ph | H | 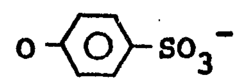 | P | S |
| 53 | Ph | 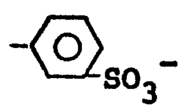 | Ph | H | $OC_3H_7$ | P | O |
| 54 | Ph | Ph | Ph | $SO_3^-$ | $OC_4H_9$ | P | O |
| 55 | Ph | Ph | Ph | $SO_3^-$ | 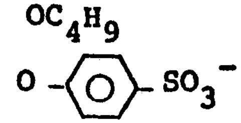 | P | O |
| 56 | Ph | Ph | Ph | H | 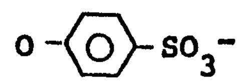 | As | O |

In this second step approximately equal molar amounts of each of the three reactants defined above are dissolved in any suitable unreactive solvent, such as toluene, tetrahydrofuran, dioxane, or other unreactive hydrocarbon solvents, and stirred while maintaining a temperature of about 0° to about 100°C., preferably room temperature, for about one-half hour to about 48 hours, preferably about three to about 20 hours, sufficient to insure complete reaction. Any suitable pressure can be used, although atmospheric pressure is preferred. The solvent can be removed from the reaction mixture in any suitable manner, for example, by distillation, including vacuum distillation, if necessary, leaving behind the novel compound defined above. On the other hand, a second solvent in which the desired product is insoluble, such as heptane, can be added to the reaction product to precipitate the novel compound therein. The novel compound can be recovered, for example, by filtration, decantation or by centrifuging.

Specific examples of nickel ylides which can be prepared by the practice of this invention are set forth in Table II.

TABLE II

| Compound | $R_1$ | $R_2$ | $R_3$ | $R_4$ | $R_5$ | $R_6$ | $R_7$ | $R_8$ | E | F | M |
|---|---|---|---|---|---|---|---|---|---|---|---|
| 1 | Ph | Ph | Ph | 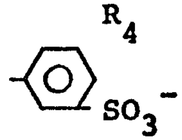 | Ph | Ph | H | Ph | P | P | O |
| 2 | 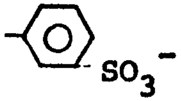 | Ph | Ph | Ph | 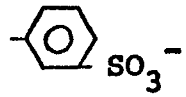 | Ph | H | Ph | P | P | O |
| 3 | Ph | Ph | Ph | Ph | Ph | 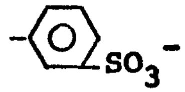 | $SO_3^-$ | Ph | P | P | O |
| 4 | Et | Et | Ph | 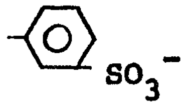 | Ph | Ph | H | Ph | P | P | O |
| 5 | Ph | Ph | Ph | Ph | 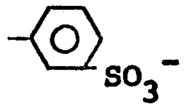 | Ph | $SO_3^-$ | Ph | P | As | O |
| 6 | Ph | Ph | Ph | Ph | Ph | 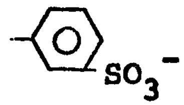 | Ph | $CH_3$ | P | As | S |
| 7 | $CH_3$ | $CH_3$ | $CH_3$ | Ph | 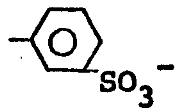 | Ph | $SO_3^-$ | $OCH_3$ | P | As | O |
| 8 | Ph | Ph | Ph | 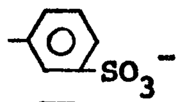 | Ph | Ph | H | $OC_3H_7$ | As | P | S |
| 9 | $CH_3$ | $CH_3$ | $CH_3$ | 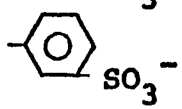 | Et | Et | H | $OC_4H_9$ | P | P | O |
| 10 | $CH_3$ | Et | Ph | 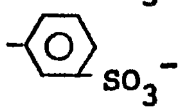 | Ph | Ph | $SO_3^-$ | 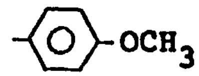 | As | P | S |
| 11 | Ph | Et | Et | Ph | 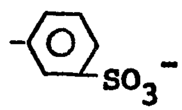 | Ph | $SO_3^-$ | $CH_3$ | As | As | S |

The following examples illustrate the invention, and are not intended to limit the invention, but rather, are presented for purposes of illustration. Example I illustrates the preparation of a nickel ylide in accordance with the practice of this invention; and Example II illustrates the use of this nickel ylide to oligomerize ethylene.

## EXAMPLE I

To 20 milliliters of 30 percent fuming sulfuric acid there were added slowly with cooling 10 grams of triphenylphosphine. The solution was then heated to 80°C. and every five minutes the solution was tested by adding one drop of the solution to water until a clear solution was obtained. The reaction mixture was cooled to room temperature, poured into 200 cc of water and neutralized with 10 percent aqueous sodium hydroxide. After setting the solution overnight at room temperature, the desired product separated by crystallization and was recovered by filtration. The recovered product, sodium diphenylphosphinobenzene-3-sulfonate has the following structure:

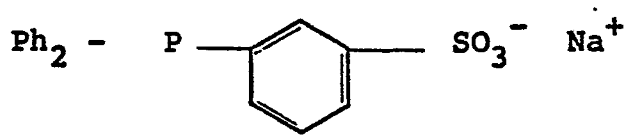

(Compound 1)

To 1.40 grams of bis(cyclooctadiene) nickel (5.1 millimoles) in 30 milliliters of toluene under an argon atmosphere there was added a solution of 1.86 grams of Compound 1 (5.1 millimoles) and 1.94 grams (5.1 millimoles) of benzoylmethylenetriphenylphosphorane:

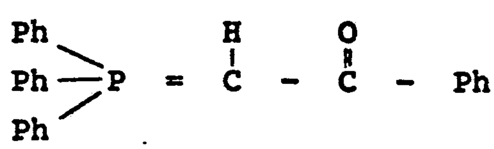

(Compound 2)

in 20 milliliters of toluene. After stirring for 18 hours at room temperature, the reaction mixture was heated to 50°C. to remove the solvent under a reduced pressure of 10 to 100 millimeters of mercury. The reaction mixture was transferred to an argon filled dry box and dissolved in toluene. Hexane was added to precipitate the product identified below as novel Compound 3. A total of 3.13 grams in 76 percent yield of the compound was recovered.

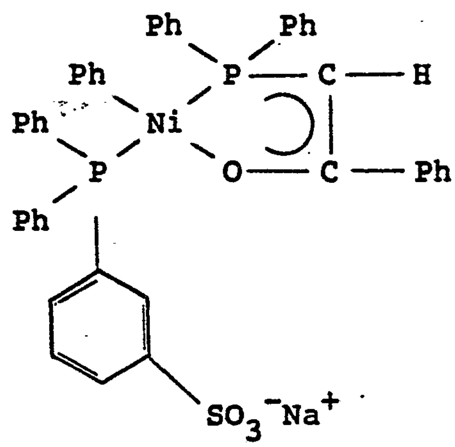

(Compound 3)

## Example II

A run was carried out wherein there was charged 0.1 millimole of the sulfonated nickel ylide catalyst obtained in Example I, Compound 3, dissolved in 100 milliliters of toluene. During the reaction precautions were taken to exclude air contamination by performing the reaction in an argon atmosphere. The reaction mixture was then heated to 50°C. and pressured with ethylene to obtain a partial pressure thereof of 200 pounds per square inch gauge (1400 kPa). The reaction mixture was stirred throughout the reaction period of two hours, during which time the temperature and pressure were maintained constant. At the end of the two-hour period the reaction mixture was cooled to room temperature and unreacted

ethylene removed therefrom by distillation. The amount of oligomer produced was determined and compared with the activity for the compound reported by the Keim et al article previously discussed. The results obtained are set forth in Table III.

TABLE III

| Run No. | Nickel Ylide Catalyst | Activity: Moles Ethylene Converted Per Mole of Nickel Catalyst |
|---|---|---|
| I | Keim et al specific catalyst | 6,000* |
| II | Compound 3 | 2,087 |

*Reported by Keim et al

An advantage of Compound 3 over that of Keim et al lies in its easy recovery from the reaction product.

Although the invention has been described in considerable detail with particular reference to certain preferred embodiments thereof, variations and modifications can be effected within the spirit and scope of the invention as described hereinbefore, and as defined in the appended claims.

Claims:

1. A process for preparing a nickel ylide of formula

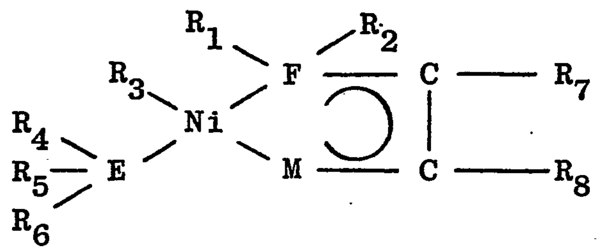

which comprises sulfonating a ligand defined by the following formula:

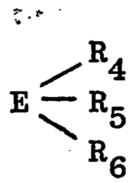

and then reacting the resulting sulfonated ligand with a zero valent nickel compound and a ylide defined by the following formula:

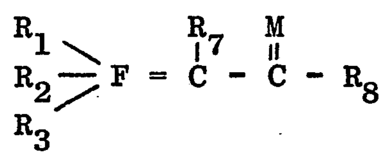

wherein $R_1$, $R_2$, $R_3$, $R_4$, $R_5$, $R_6$, $R_7$ and $R_8$, alike or different, are selected from hydrogen, alkyl radicals having from one to 24 carbon atoms, aryl radicals having from six to 20 carbon atoms, alkenyl radicals having from two to 30 carbon atoms, cycloalkyl radicals having from three to 40 carbon atoms, aralkyl and alkaryl radicals having from six to 40 carbon atoms, halogen radicals, hydroxyl, alkoxy, or aryloxy groups, hydrocarbyl groups carrying halogen, hydroxyl, alkoxy or aryloxy, and a sulfonato group or an alkyl, aryl, alkenyl, cycloalkyl, aralkyl or alkaryl group carrying a sulfonato group, provided that at least one sulfonato group

is located in $R_4$, $R_5$ and $R_6$ and at least one of $R_4$, $R_5$ and $R_6$ is an aryl group; M is sulfur or oxygen; E is phosphorus, arsenic, antimony or nitrogen; and F is phosphorus, arsenic or antimony.

2. A process as claimed in claim 1 wherein E and F are both phosphorus and M is oxygen.

3. A process as claimed in claim 1 or claim 2 wherein each of $R_4$, $R_5$ and $R_6$ is phenyl.

4. A process as claimed in claim 1 or 2 or 3 wherein each of $R_1$, $R_2$, $R_3$ and $R_8$ is phenyl and $R_7$ is hydrogen.

5. A process as claimed in any preceding claim wherein said sulfonating is conducted using $SO_3$ in the presence of a strong mineral acid.

6. A process as claimed in claim 5 wherein said sulfonating is conducted using fuming sulfuric acid.

7. A process as claimed in claim 6 wherein said fuming sulfuric acid is used in an amount of at least one mole per mole of ligand.

8. A process as claimed in claim 7 wherein said fuming sulfuric acid is used in an amount of from two to 20 moles per mole of ligand.

9. A process as claimed in any of claims 5 to 8 wherein the reactants during said sulfonating are contacted at a temperature of from 0° to 200°C for from one minute to 38 hours.

10. A process as claimed in claim 9 wherein the reactants during said sulfonating are contacted at a temperature of from 40° to 100°C for from 30 minutes to four hours.

11. A process as claimed in any preceding claim wherein the sulfonated ligand is reacted with said zero valent nickel compound and said ylide at a temperature of from 0° to 100°C for from one-half to 48 hours.

12. A process as claimed in claim 11 wherein the sulfonated ligand is reacted with said zero valent nickel compound and said ylide at about room temperature for from three to 20 hours.

13. A process as claimed in any preceding claim wherein said zero valent nickel compound is bis(cyclooctadiene) nickel.